# EUROPÄISCHE PATENTANMELDUNG

(11) **EP 0 616 815 A1**
(43) Veröffentlichungstag der Anmeldung: **28.09.1994**
(21) Anmeldenummer: 94101783.2
(22) Anmeldetag: 07.02.1994
(51) Int. Cl.: A61M 1/00

(54) **Einrichtung zum Einstellen des Vakuums in einer evakuierten Wundsaugdrainageflasche**

(30) Priorität: 24.03.1993 CH 889/93
(71) Anmelder: Genossenschaft VEBO Solothurnische Eingliederungsstätte für Behinderte, CH-4702 Oensingen (CH)
(72) Erfinder: Müller, Walter, CH-4703 Kestenholz (CH); Nützi, Silvan, CH-4855 Wolfwil (CH); Born, Alessandro, CH-4702 Oensingen (DE)
(74) Vertreter: Fillinger, Peter, Dr.

(57) **Zusammenfassung**

Die Einrichtung dient dem Einstellen des Vakuums in einer evakuierten Wundsaugdrainageflasche (2) mit einem Anschlussstück (4) für eine Drainageleitung. Zur raschen Einstellung des gewünschten Unterdrucks in der Saugdrainageflasche ist ein Ventilkörper (3) mit einem Ventil (15, 16) vorgesehen, der an eine Saugdrainageflasche einerseits und an ein Einstellgerät (5) anderseits anschliessbar ist. Das Einstellgerät weist ein Druckmessgerät (27) auf und ist mit Mitteln ausgestattet, welche das Ventil (15, 16) mit dem Druckmessgerät (27) verbinden und wahlweise das Ventil (15, 16) öffnen.

## Beschreibung

Die vorliegende Erfindung bezieht sich auf eine Einrichtung gemäss dem Oberbegriff des Anspruchs 1.

Wundsaugdrainageflaschen zur postoperativen Wundbehandlung sind bekannt und beispielsweise in der EP-PS 288 679 sowie in den CH-PS'n 596 840 und 584 037 beschrieben. Solche Saugdrainageflaschen haben - soweit sie in einem Autoklaven sterilisiert und evakuiert sind - ein zwar gleichmässiges Vakuum; nachteilig indessen ist, dass dieses Vakuum für bestimmte Anwendungsfälle wie zum Beispiel nach Hand- oder Gehirnoperationen zu gross ist.

Die vorliegende Erfindung stellt sich daher die Aufgabe, eine Einrichtung zu schaffen, die es ermöglicht, an Saugdrainageflaschen mit zu hohem Vakuum eine geringere Druckdifferenz in feststellbarer Grösse herzustellen.

Erfindungsgemäss wird diese Aufgabe gelöst durch die kennzeichnenden Merkmale des Anspruchs 1.

Die Erfindung hat den Vorteil, dass das gewünschte Vakuum mit einem einzigen Knopfdruck und nicht in mehreren sich wiederholenden Betätigungsschritten herbeigeführt werden kann.

Anhand der beiliegenden schematischen Zeichnung wird die Erfindung beispielsweise erläutert.

Die Hinweisziffer 1 bezeichnet einen Stopfen, der auf den Kragen einer Saugdrainageflasche 2 gebräuchlicher Art aufgesetzt ist und diese verschliesst. Der Stopfen 2 ist hinsichtlich Aufbau und Funktionsweise eingehend in der EP-PS 288 679 beschrieben. Auf seine Beschreibung wird daher vorliegend verzichtet. In der in einem Autoklaven bei einer Temperatur von zirka 130° und einem Druck von 3,5 bar sterilisierten und evakuierten Saugdrainageflasche 2 herrscht in atmosphärischer Umgebung ein Unterdruck von 0,1 bar. Ist in der Saugdrainageflasche ein kleinerer Unterdruck von z.B. 0,5 bar erwünscht, muss ein Ventilkörper 3 auf den Anschlussschlauch 4 aufgesteckt und eine allfällig vorhandene Schlauchklemme gelöst werden. (Das Aufstecken des Ventilkörpers 3 auf den Anschlussschlauch erfolgt jedoch in der Regel vor der Sterilisation und Evakuation im Autoklaven.) Alsdann wird das Einstellgerät 5 auf den Ventilkörper 3 aufgesteckt und betätigt, bis sich an dessen Druckanzeiger 6 der gewünschte Unterdruck in der Saugdrainageflasche ablesen lässt.

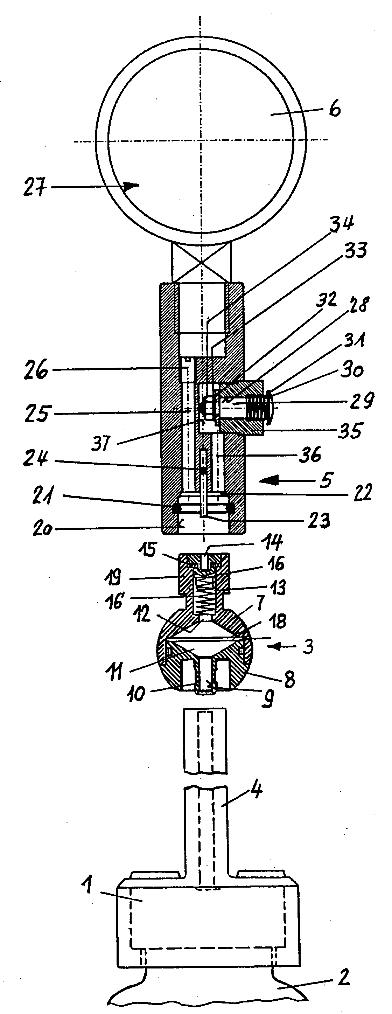

Die aus dem Ventil 3 und dem Einstellgerät 5 bestehende Einrichtung wird nachfolgend anhand der Fig. 2 beschrieben.

Der Ventilkörper 3 weist zwei mit Pressitz dicht verbundene Teile 7 und 8 auf, die einen axial orientierten Strömungskanal 9 bilden. Der untere Teil 8 ist mit einem Schlauchnippel 10 versehen, auf den der Anschlussschlauch 4 einer Saugdrainageflasche 2 zu stecken ist. Anschliessend an den Schlauchnippel 10 weitet sich der Strömungskanal 9 zu einer nach oben und unten konisch verjüngten Kammer 11, an die eine Verbindungsbohrung 12 zu einer Ventilkammer 13 anschliesst. Die Ventilkammer 13 ist durch eine Entlüftungsbohrung 14 mit dem Einstellgerät 5 bzw. der Aussenatmosphäre verbindbar. Die Entlüftungsbohrung 14 ist von einer einen Ventilsitz 15 bildenden Dichtung umgeben, gegen die eine Ringrippe eines Ventilkörpers 16 bei geschlossenem Ventil dicht anliegt. Eine vorgespannte Ventilfeder 16' drückt den Ventilkörper 16 auf den Ventilsitz 15 bzw. hält ihn in seiner Schliessstellung.

Die Ventilfeder 16' ist so dimensioniert, dass sie erst bei einem Überdruck von 1,5 bar öffnet. Daher ist der Ventilkörper 3 während einer Dampfsterilisation im Autoklaven vorzugsweise auf den Schlauch 4 aufgesteckt, da der Heissdampf bei Drucken über 1,5 bar in die Saugdrainageflasche 2 eintreten und das Flascheninnere sterilisieren kann, worauf sich - nach beendigter Sterilisation - beim Abkühlen der Flasche im Flascheninneren ein Vakuum bildet.

Zwischen dem oberen und unteren Teil 7 bzw. 8 des Ventilkörpers 3 ist ein beidseits durch Gitterplatten 18 gestütztes Sterilfiltervlies 17 eingespannt, das die Kammer 11 unterteilt. Die lichte Weite der Kammer 11 muss möglichst gross sein, damit der Strömungswiderstand im Strömungskanal 9 durch das eingesetzte Sterilfiltervlies 17 nicht vergrössert wird.

Sterilfilter sind bekannt und beispielsweise unter der Bezeichnung Mikrofilterlaminat GSC-Nr. GO8263 auf dem Markt erhältlich (Firma W. L. Gove & Associates GmbH, D-8011 Putzbrunn). Das obere Ende des Ventilkörpers 3 ist als zylindrischer Stecker 19 gestaltet, der in eine mit einer Ringdichtung 21 versehene Muffe 20 des Einstellgerätes 5 steckbar ist. In die Muffe 20 ragt axial ein Stift 23, der längsverschieblich und mittels einer Spannschraube 24 feststellbar ist. Der Durchmesser des Stiftes 23 ist kleiner als jener der Entlüftungsbohrung 14. Wird der Stecker 19 in die Muffe 20 geschoben, kommt seine zylindrische Aussenfläche zur Anlage mit der Ringdichtung 21 und gleichzeitig schiebt sich der Stift 23 in die Entlüftungsbohrung 14, drückt den Ventilkörper 16 vom Ventilsitz 15 weg und öffnet das Ventil.

Ein mit einer Drossel 26 versehener Ausgleichskanal 25 verbindet die Muffe 20 mit dem Druckmessgerät 27. Hierdurch zeigt der Druckanzeiger 6 des Druckmessgerätes 27 nach dem Aufstecken des Einstellgerätes 5 auf den Ventilkörper 3 den in der Saugdrainageflasche 2 herrschenden Druck an.

Durch die verstellbare Drossel 26 wird sicher gestellt, dass zwischen der Flasche 2 und dem Druckmessgerät kontinuierlich ein Druckausgleich stattfindet. Zwischen der Muffe 20 und dem Ausgleichskanal 25 ist ein Ringkanal 22 ausgespart, der bei in die Muffe 20 eingeschobenem Stecker 19 frei bleibt. Diametral in das Einstellgerät 5 erstreckt sich Belüftungskammer 37, die nach aussen durch einen im Querschnitt ringförmigen Ventilsitzkörper 35 abgeschlossen ist. Der Ventilsitzkörper 35 ist achsial von einem Lufteinlasskanal 28 durchsetzt, in welchem ein mit einem Druckknopf 30 versehener Schieber 29 achsial verschiebbar gelagert ist. Der Schieber 29 überragt in seiner Ruhelage mit dem Druckknopf 30 das Einstellgerät 5 und ist auf der Druckknopfseite achsial von einer auf Druck vorgespannten Schraubenfeder 31 versehen, die einerseits gegen den Druckknopf 30 und anderseits gegen eine entsprechende Ringnut im Ventilsitzkörper 35 abgestützt ist. Auf das innere, mit einem Aussengewinde versehene Ende des Schiebers 29 sind ein Dichtungsring 32 und eine Unterlagsscheibe 33 achsial aufgesetzt und mit einer Mutter 34 festgehalten. Die Feder 31 hält bei unbelastetem Druckknopf 30 den mit der Unterlagsscheibe 33 und der Mutter 34 festgehaltenen Dichtungsring 32 in dichtender Anlage mit dem Ventilsitzkörper 35 und sperrt den Lufteinlasskanal 28. In dieser in der Zeichnung gezeigten Ruhestellung des Schiebers 29 ist die Belüftungskammer gegen die Aussenatmosphäre abgedichtet. Die Belüftungskammer 37 kommuniziert über einen Belüftungskanal 36 und die Ringkammer 22 mit dem Ausgleichskanal 25. Sind das Ventil 3 und das Einstellgerät 5 auf den Schlauch 4 einer Saugdrainageflasche 2 aufgesteckt, ist das Druckmessgerät 27 ausschliesslich vom Innendruck der Saugdrainageflasche 2 beaufschlagt.

Soll die Druckdifferenz zwischen dem Innern der Saugdrainageflasche 2 und der Aussenatmosphäre verringert werden, wird auf den Druckknopf 30 gedrückt und der Dichtungsring 32 vom Ventilsitzkörper 35 abgehoben. In diesem Moment kann die Aussenluft zwischen dem Schieber 29 und der Wand des Belüftungskanals 28 in den Belüftungskanal 36 und von dort in die Saugdrainageflasche 2 strömen. Da auch bei gedrücktem Druckknopf 30 das Innere der Saugdrainageflasche 2 über den Ausgleichskanal 25 in Verbindung mit dem Druckmessgerät 27 steht zeigt das Druckmessgerät 27 den steigenden Innendruck in der Saugdrainageflasche 2 kontinuierlich an. Ist die gewünschte Druckdifferenz erreicht, lässt man den Druckknopf 30 frei, worauf er unter der Wirkung der Feder 31 in die Ruhelage zurückkehrt, in der der Lufteinlasskanal 28 wieder gegen aussen gesperrt ist. Danach kann der Schlauch 4 einer Saugdrainageflasche von der Einrichtung 3, 5 getrennt und mit einem Wunddrainageschlauch verbunden werden.

## Patentansprüche

1. Einrichtung zum Einstellen des Vakuums in einer evakuierten Wundsaugdrainageflasche (2) mit einem Anschlussstück (4) für eine Drainageleitung, gekennzeichnet durch einen Ventilkörper (3), der von einem Strömungskanal (9) durchsetzt ist und der am ersten Ende des Strömungskanals (9) ein zum Anschlussstück (4) der Saugdrainageflasche (2) komplementäres Anschlussstück (10) bildet und am zweiten Ende ein Kupplungsteil (19) für den lösbaren Anschluss an ein Einstellgerät (5) aufweist, durch ein den Strömungskanal (9) zwischen seinen beiden Enden sperrendes Ventil (15, 16), das den Strömungskanal (9) frei gibt, wenn am zweiten Ende der Druck einen zwischen dem Atmosphärendruck und einem höheren Sterilisationsdruck liegenden Grenzwert übersteigt, wobei das Einstellgerät (5) durch folgende Merkmale charakterisiert ist:
- ein zum Kupplungsteil (19) des Ventilkörpers (3) komplementärer Kupplungsteil (20);
- ein Druckmessgerät (27);
- ein das komplementäre Kupplungsteil (20) mit dem Druckmessgerät (27) verbindender, mit einer Drossel (26) versehener Ausgleichskanal (25);
- Mittel (22, 28 bis 37) um den Ausgleichskanal (25) wahlweise mit der Umgebungsatmosphäre zu verbinden oder gegen diese abzusperren und
- ein Öffnungselement (23), um bei eingekuppelten Kupplungselementen (19, 20) das Ventil (15, 16) in eine Offenstellung zu zwingen.

2. Einrichtung nach Anspruch 1, dadurch gekennzeichnet, dass im Strömungskanal (9) ein Sterilfilter (17) angeordnet ist.

3. Einrichtung nach Anspruch 2, dadurch gekennzeichnet, dass der lichte Querschnitt des Strömungskanals (9) im Bereich des Sterilfilters (17) ein Mehrfaches des kleinsten Strömungsquerschnitts im Strömunskanal (9) beträgt.

4. Einrichtung nach einem der Ansprüche 1 bis 3, dadurch gekennzeichnet, dass der Strömungskanal (9) am zweiten Ende eine von einem Ventilsitz (15) umgebene Bohrung (14) aufweist, und dass ein von einer Rückstellkraft beaufschlagter Ventilkörper (16) mit einer gegen den Ventilsitz (15) liegenden Ringrippe die Bohrung (14) abschliesst.

5. Einrichtung nach einem der Ansprüche 1 bis 4, dadurch gekennzeichnet, dass die beiden Kupplungsteile (19, 20) eine druckdichte Steckkupplung bilden.

6. Einrichtung nach Anspruch 5, dadurch gekennzeichnet, dass das Öffnungselement (23) ein axial im komplementären Kupplungsteil (20) angeordneter Stift ist, dessen Durchmesser kleiner als jener des zweiten Endes (14) des Strömungskanals (9) ist und der bei verbundenen Kupplungsteilen (19, 20) den Ventilkörper (16) vom Ventilsitz (15) abhebt.

7. Einrichtung nach Anspruch 6, dadurch gekennzeichnet, dass der Stift (23) axial verstellbar im Einstellgerät (5) befestigt ist.

8. Einrichtung nach Anspruch 1, dadurch gekennzeichnet, dass die Mittel (22, 28 bis 37) einen den Ausgleichskanal (25) mit der Aussenatmosphäre verbindenden Belüftungspfad (22, 28, 36, 37) aufweisen, in dem ein federbelasteter Schieber (29) zwischen einer Ruhe- und einer Arbeitslage verschiebbar ist, wobei der Schieber (29) in der Ruhelage das Einstellgerät (5) in Form eines Druckknopfes (30) überragt und den Belüftungspfad (22, 28, 36, 37) gegen die Aussenatmosphäre sperrt, und dass der Schieber (29) in der Arbeitslage die Feder (31) spannt und den Belüftungspfad (22, 28, 36, 37) mit der Umgebungsatmosphäre verbindet.
